# EUROPEAN PATENT APPLICATION

(11) **EP 1 710 243 A1**
(43) Date of publication of application: **11.10.2006**
(21) Application number: 05709376.7
(22) Date of filing: 27.01.2005
(51) Int. Cl.: C07D 401/12, A61K 31/4375, A61P 9/00, A61P 43/00

(54) **THERAPEUTIC AGENT FOR VASOSPASM ACCOMPANYING BYPASS OPERATION**

(30) Priority: 29.01.2004 JP 2004020882
(71) Applicant: Asahi Kasei Pharma Corporation, Tokyo 101-8481 (JP)
(72) Inventor: SHIMOKAWA, Hiroaki, 8160863 (JP)
(74) Representative: Forstmeyer, Dietmar
(86) International application number: PCT/JP2005/001102
(87) International publication number: WO 2005/073218

(57) **Abstract**

An agent and composition for the prevention of and treatments for vasospasm accompanying a bypass operation, which each contains as an active ingredient either a compound represented by the general formula (I) or an acid addition salt or hydrate thereof. (In the formula, R¹ represents hydrogen or hydroxy). The agent and composition for the prevention of and treatments for vasospasm accompanying a bypass operation is effective in the prevention of or treatments for vasospasm accompanying a bypass operation.

## Description

### Technical Field

The present invention relates to an agent and a composition for prevention of and treatment for vasospasm accompanying a bypass operation.

### Background Art

An arterial bypass operation is performed when occlusion or stenosis of an artery has occurred. Upon the arterial bypass operation, arterial spasm may occur during or after a coronary artery bypass grafting. The arterial spasm is characterized by being more intense and persistent than the arterial spasm that occurs during a nonoperative period. Further, the arterial spasm hardly responds to a nitro-compound or a calcium antagonist which has been administered after the occurrence of the arterial spasm, resulting in a large clinical problem in the bypass operation (Non-patent Document 1).
Meanwhile, a compound represented by the general formula (I) has an inhibitory activity against kinases such as Rho kinase, myosin light chain kinase or protein kinase C, and represents a relaxing effect on vascular smooth muscles, an increasing effect on blood flow, a reducing effect on blood pressure, a protecting effect on a brain or heart, or the like. Thus, it is known that the compound represented by the general formula (I) serves as an effective substance for a vasodilating agent (especially, for a therapeutic agent for angina pectoris), a therapeutic agent for hypertension, a protecting agent for a brain or heart, a therapeutic agent for arteriosclerosis (see, for example, Patent Documents 1 to 9 and Non-patent Documents 2 to 5).

However, the arterial spasm during the bypass operation is refractory to treatment with a common vasodilating agent including the nitro-compound and the calcium antagonist, and therefore the vasodilating agent is not necessarily effective in the prevention of and treatment for the vasospasm accompanying the by pass operation. The description has not been reported which suggests that: the compound represented by the general formula (I) is effective in the prevention of and treatment for the vasospasm accompanying the bypass operation; and a composition for the prevention of and treatment for the vasospasm accompanying the bypass operation, which contains the compound represented by the general formula (I) and at least one pharmaceutically acceptable therapeutic agent selected from the calcium antagonists and nitro-compounds is effective in the prevention of and treatment for the vasospasm accompanying the bypass operation.

Patent Document 1: JP-A-61-152658
Patent Document 2: JP-A-61-227581
Patent Document 3: JP-A-02-256617
Patent Document 4: JP-A-04-264030
Patent Document 5: JP-A-06-056668
Patent Document 6: JP-A-06-080569
Patent Document 7: JP-A-07-80854
Patent Document 8: WO 98/06433
Patent Document 9: WO 00/03746

Non-patent Document 1: Hiroshi Hayafuji, "Separate volume, Nippon Rinsho, Series: Syndromes in separate regions No. 12, Syndromes in Circulatory Organs I, including other Circulatory Diseases", First Edition, Nippon Rinsho Corporation, August 30, 1996, p.667-671
Non-patent Document 2: Br. J. Pharmacol. 98, 1091 (1989)
Non-patentDocument3: J. Pharmacol. Exp. Ther. 259, 738 (1991)
Non-patent Document 4: Circulation 96, 4357 (1997)
Non-patent Document 5: Cardiovasc. Res., 43, 1029 (1999)

### Disclosure of the Invention

### Problem to be solved by the Invention

The present invention has been made in view of the above circumstances and provides a medicine for the prevention of or treatment for vasospasm accompanying a bypass operation. Means for solving the Problem

The inventors of the present invention have made extensive studies on a compound represented by the general formula (I) or an acid addition salt or a hydrate thereof. As a result, they have found that the compound has a preventing/treating effect on the vasospasm accompanying the bypass operation, which may not be expected from the known effects such as a relaxing effect on vascular smooth muscles, an increasing effect on blood flow, a reducing effect on blood pressure, a protecting effect on a brain or heart, and the like.

That is, the present invention includes:
(1) an agent for prevention of and/or treatment for vasospasm accompanying a bypass operation, which comprises as an active ingredient either a compound represented by the following general formula (I): 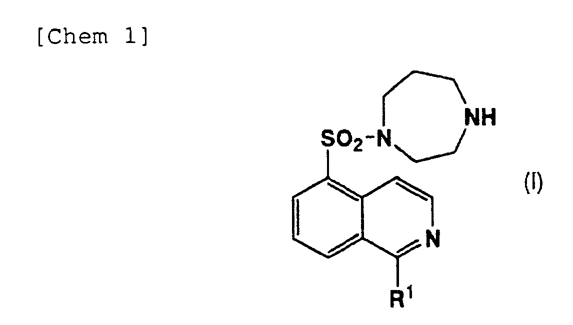 where R¹ represents a hydrogen atom or a hydroxyl group, or an acid addition salt or hydrate thereof;
(2) the agent for prevention of and/or treatment for vasospasm accompanying a bypass operation according to the above item (1), wherein the bypass operation comprises coronary artery bypass grafting;
(3) the agent for prevention of and/or treatment for vasospasm accompanying a bypass operation according to the above item (1) or (2), wherein the vasospasm comprises vasospasm which does not respond to a calcium antagonist and/or a nitro-compound;
(4) the agent for prevention of and/or treatment for vasospasm accompanying a bypass operation according to any one of the above items (1) to (3), wherein the vasospasm occurs in a region which differs from an anastomosis region.

The compound represented by the general formula (I) of the present invention can be synthesized in accordance with a known method described in, for example, Chem. Pharam. Bull., 40 (3) 770-773 (1992), JP-A-61-152658, or the like. Further, an acid addition salt of the compound represented by the general formula (I) of the present invention is preferably a pharmaceutically acceptable nontoxic salt. Examples of the salt include: salts of inorganic acids such as hydrochloric acid, hydrobromic acid, phosphoric acid, and sulfuric acid; and salts of organic acids such as acetic acid, citric acid, tartaric acid, lactic acid, succinic acid, fumaric acid, maleic acid, and methanesulfonic acid. Furthermore, examples of a hydrate of the compound represented by the general formula (I) of the present invention include a 1/2 hydrate, a 1 hydrate, and a 3 hydrate.

When an agent for the prevention of and treatment for vasospasm accompanying a bypass operation of the present invention is prepared as a formulation having a form suitable for administration, the compound represented by the above general formula (I), or an acid addition salt thereof or a hydrate may be mixed with a known medicamentary acceptable carrier. Examples of the carrier include: gelatin; sugars such as lactose and glucose; starches such as wheat starch, rice starch, and corn starch; fatty acids such as stearic acid; fatty acid salts such as calcium stearate and magnesium stearate; talc; vegetable oils; alcohols such as stearic alcohol and benzyl alcohol; gum; and polyalkylene glycol.

In addition, examples of a liquid carrier generally include: water; a physiological saline; dextrose or a similar sugar solution; and glycols such as ethylene glycol, propylene glycol, polyethylene glycol, and polypropylene glycol. When a capsule is prepared, it is generally preferred to use gelatin.

An exemplified agent of the present invention for the prevention of and treatment for the vasospasm accompanying the bypass operation, which is composed of the above-mentioned carrier and the compound represented by the general formula (I), or an acid addition salt or hydrate thereof, contains generally 0.01% by weight or more, and 80% by weight or less, and preferably 60% by weight or less of the active ingredient.

An administration method includes an oral administration or a parenteral administration. Examples of dosage forms suitable for the oral administration include a tablet, a capsule, a powder, a granule, a liquid formulation, and an elixir, and an example of a dosage form suitable for the parenteral administration includes a liquid formulation.
When the parenteral administration is performed through an intramuscular injection, an intravenous injection, or a subcutaneous injection, the agent of the present invention is administered in a form of an sterile solution in which sodium chloride or other solute such as glucose is added in order to make the compound represented by the general formula (I) or the acid addition salt or hydrate thereof to be isotonic.

When the administration is performed through an injection, the agent of the present invention is also preferably dissolved in a sterile water, a solution of lidocaine hydrochloride (for an intramuscular injection), a physiological saline, a solution of glucose, a solution for an intravenous injection, an electrolytic solution (for an intravenous injection), or the like. In such dissolution, the solution may be adjusted to contain generally 0.01% by weight or more and 20% by weight or less, and preferably 0.1% by weight or more and 10% by weight or less of the active ingredient. In a case of a liquid formulation for oral administration, a preferable example includes a suspension or a syrup containing 0.01% to 20% by weight of the active ingredient. In this case, a aqueous excipient such as a flavor, a syrup, or a pharmaceutical micelle is exemplified as the carrier.

A dose of the agent of the present invention for the prevention of and treatment for vasospasm accompanying the bypass operation varies depending on, for example: the age, health condition, body weight, or degree of symptoms of a subject to be administered; the kind or treatment frequency of other treatment, if it is simultaneously performed; or properties of the desired effect, or the administration route or administration schedule. However, in general, the agent of the present invention is administered in 0.01 to 20 mg/kg per day for parenteral administration and 0.02 to 100 mg/kg per day for oral administration.

The medicine of the present invention to be used for the treatment for and/or prevention of the vasospasm accompanying the bypass operation can be appropriately used in combination with other one or more drugs (hereafter, the drug to be used in combination with a medicine containing as an active ingredient the compound represented by the general formula (I) or the acid addition salt or hydrate thereof is referred to as a "concomitant drug"). Decision of whether the use of the concomitant drug is preferable or not is understood by confirming that a medicine containing as an active ingredient the compound represented by the general formula (I) or the acid addition salt or hydrate thereof shows a more preferable result when it is administered in combination with a concomitant drug than in a case where the compound represented by the general formula (I) is administered alone.

Examples of the concomitant drug include: calcium antagonists , nitro-compounds, other coronary vasodilators, and catecholamines. More specific examples of the drug include calcium antagonists (such as diltiazem, verapamil, amlodipine, efonidipine, nisoldipine, nitrendipine, nifedipine, benidipine, nicardipine, aranidipine, cilnidipine, barnidipine, felodipine, manidipine, nilvadipine, azelnidipine, and salts thereof), nitro-compounds (such as isosorbide dinitrate, nitroglycerin, isosorbide mononitrate, amyl nitrite, and sodium nitroprusside), other coronary vasodilators (such as nicorandil, dilazep, etafenone, trapidil, trimetazidine, and salts thereof), and catecholamines (dopamine, dobutamine, norepinephrine, epinephrine, phenylephrine, methoxamine, etilefrine, denopamine, docarpamine, isoprenaline, and salts thereof).

Upon the combination, the timing of the administration of each of the medicine containing as an active ingredient the compound represented by the general formula (I) or the acid addition salt or hydrate thereof and the concomitant drug is not limited. They may be administered simultaneously, or they may be administered at a time interval as long as their effects are expected. Therefore, the medicine containing as an active ingredient the compound represented by the general formula (I) or the acid addition salt or hydrate thereof and the concomitant drugmay be prepared in separate forms, or they may be mixed to have a form of one medical composition. Meanwhile, they may be administered through the same administration route, or each of them may be administered through a different administration route. When the medical composition containing both is to be prepared, the compounding ratio between the compound of the present invention or the pharmacologically acceptable salt and the concomitant drug, the form obtained after mixing both, or the like can be appropriately determined depending on the object of the administration, the administration route, the objective disease, the symptoms, the properties of the agent, ease of the administration, or the like.
When the vasospasm accompanying the bypass operation is to be prevented or treated by concomitantly using the concomitant drug, the dose of the concomitant drug (such as a calcium antagonist or a nitro-compound) is exemplified by 1/500 to 1 with respect to the case where the dose of the compound represented by the general formula (I) or the acid addition salt or hydrate thereof is 1.

### Effect of the Invention

According to the present invention, there is provided an agent or a composition for the prevention of and treatment for the vasospasm accompanying the bypass operation.

### Brief Description of the Drawing

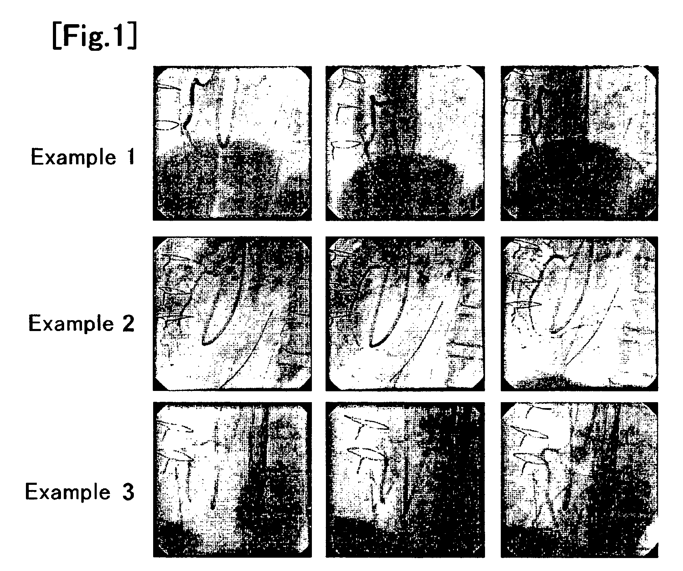

[FIG. 1] FIG. 1 shows angiographic photographs of the right coronary artery A: at the occurrence of vasospasm, B: after the administration of isosorbide dinitrate into the coronary artery, and C: after the administration of fasudil hydrochloride into the coronary artery, of the following examples 1 to 3, respectively.

### Best Mode for carrying out the Invention

Hereinafter, the present invention will be described in more detail by referring to the examples and reference examples. However, the present invention is not limited to them.

### Example 1

To a patient who had been subj ected to off pump beating coronary artery bypass grafting (in which a left internal thoracic artery was anastomosed to each of a left anterior descending coronary artery and a diagonal branch), during and after the operation, isosorbide dinitrate (2 to 5 mg/hour), diltiazem (1 to 2 mg/hour), nicorandil (4 to 6 mg/hour), and catecholamines (1 to 2 "g/kg per minute of dopamine and 1 to 2 "g/kg per minute of dobutamine) were continuously intravenously administered. However, after three hours of the operation, severe coronary vasospasm occurred. The coronary vasospasm occurred in a right coronary artery which was not related to the bypass operation. Isosorbide dinitrate (total of 10 mg) was administered into the coronary artery, but it had no effect on the vasospasm. Therefore, fasudil hydrochloride was dissolved in a physiological saline and then the mixture was administered into the right coronary artery for 15 minutes at a rate of 1.5 mg/minute. As a result, the coronary vasospasm was resolved (see FIG. 1). At the occurrence of the vasospasm and after the administration of isosorbide dinitrate, the right coronary artery became thin like a thread due to the vasospasm and peripheral part thereof was not illustrated. However, after the administration of fasudil hydrochloride, the peripheral part was illustrated. Thus, it was found that the vasospasm had been resolved. After that, fasudil hydrochloride was dissolved in the physiological saline and the mixture was continuously administered into the vein for 72 hours (tapered from 10 mg/hour). The coronary vasospasm did not occur again.

### Example 2

To a patient who had been subj ected to off pump beating coronary artery bypass grafting (in which a saphenous vein graft was anastomosed from an aorta to a left anterior descending coronary artery), during and after the operation, isosorbide dinitrate (1 to 5 mg/hour), diltiazem (1 to 2 mg/hour), and catecholamines (1 to 2 "g/kg per minute of dopamine, dobutamine, and norepinephrine, respectively) were continuously intravenously administered. However, after five hours of the operation, severe coronary vasospasm occurred. The coronary vasospasm occurred in a right coronary artery and a left circumflex coronary artery which were not related to the bypass operation. Isosorbide dinitrate (total of 10 mg) was administered into the right coronary artery, but it had no effect on the vasospasm. Therefore, fasudil hydrochloride was dissolved in a physiological saline and then the mixture was administered into the right coronary artery for 15 minutes at a rate of 1.5 mg/minute. As a result, the coronary vasospasm was resolved (see FIG. 1). At the occurrence of the vasospasm and after the administration of isosorbide dinitrate, the distal part thereof from the portion where the vasospasm had occurred was not illustrated. However, after the administration of fasudil hydrochloride, the peripheral part was illustrated. Thus, it was found that the vasospasm had been resolved. After that, fasudil hydrochloride was dissolved in the physiological saline and the mixture was continuously administered into the vein for 72 hours (tapered from 10 mg/hour). The coronary vasospasm did not occur again.

### Example 3

To a patient who had been subj ected to off pump beating coronary artery bypass grafting (in which a right internal thoracic artery was anastomosed to a left anterior descending coronary artery and a left internal thoracic artery was anastomosed to a posterolateral branch of a left circumflex coronary artery), during the operation, isosorbide dinitrate (1 to 3 mg/hour), diltiazem (0.5 to 1mg/hour), nicorandil (2 to 3 mg/hour), and catecholamines (1 to 5 "g/kg per minute of dopamine, 1 to 2 "g/kg per minute of dobutamine, and 1 to 2 "g/min of norepinephrine) were continuously intravenously administered. However, severe coronary vasospasm occurred immediately thereafter. The vasospasm occurred in the right coronary artery which was not related to the bypass operation and the bypass artery grafts (the right internal thoracic artery and the left internal thoracic artery) while the anastomosis region was left open. For the vasospasm, isosorbide dinitrate (total of 20 mg) was administered into the coronary artery and into the grafts, but it had no effect on the vasospasm. Therefore, fasudil hydrochloride was dissolved in a physiological saline and then the mixture was administered into each of the blood vessels for 15 minutes at a rate of 1.5 mg/minute. As a result, the coronary vasospasm was resolved (see FIG. 1). At the occurrence of the vasospasm and after the administration of isosorbide dinitrate, distal part from the region where the vasospasm had occurred was not illustrated. However, after the administration of fasudil hydrochloride, the peripheral part was illustrated. Thus, it was found that the vasospasm had been resolved. After that, fasudil hydrochloride was dissolved in the physiological saline and the mixture was continuously administered into the vein for 48 hours (tapered from 30 mg/hour). The coronary vasospasm did not occur again.

### Example 4

An acute toxicity test with the compound of the present invention was performed on rats (Jcl : Wistar, 5-weeks-old) and mice (Slc: ddY, 5-weeks-old). As a result, the compound was found to have low toxicity. Table 1 shows the result.

**[Table 1]**

| Compound | Animal species | Administration route | Sex | Result |
|---|---|---|---|---|
| (General formula I) | | | | LD₅₀ (mg/kg) |
| R₁=H | Rat | Intravenous | Male | 59.9 |
| | | | Female | 63.9 |
| | | Oral | Male | 335.0 |
| | | | Female | 348.0 |
| | | Subcutaneous | Male | 123.2 |
| | | | Female | 128.3 |
| R₁=H | Mouse | Intravenous | Male | 63.7 |
| R₁=OH | Mouse | Intravenous | Male | 119.3 |

### Example 5

Preparation example (sterile injection)
Ingredients shown in the following Table 2 were dissolved in distilled water for injection and then distilled water for injection was additionally added thereto to make it a required final mass. Two ml of this solution was sealed within an ampule and the whole was subjected to heat sterilization.

**[Table 2]**

| | Ingredients | Amounts |
|---|---|---|
| 10 mg preparation | Hydrochloride of the general formula (I) (where R¹ represents a hydrogen atom) | 10 mg |
| | Sodium hydrochloride | 16 mg |
| | Distilled water | Adequate amount Total volume was made to be 2 ml |
| 30 mg preparation | Hydrochloride of the general formula (I) (where R¹ represents a hydrogen atom) | 30 mg |
| | Sodium hydrochloride | 16 mg |
| | Distilled water | Adequate amount Total volume was made to be 2 ml |
| 60 mg preparation | Hydrochloride of the general formula (I) (where R¹ represents a hydrogen atom) | 60 mg |
| | Sodium hydrochloride | 16 mg |
| | Distilled water | Adequate amount Total volume was made to be 2 ml |
| 10 mg preparation | Hydrochloride of general formula (I) (where R¹ represents a hydroxyl group) | 10 mg |
| | Sodium hydrochloride | 16 mg |
| | Distilled water | Adequate amount Total volume was made to be 2 ml |
| 30 mg preparation | Hydrochloride of general formula (I) (where R¹ represents a hydroxyl group) | 30 mg |
| | Sodium hydrochloride | 16 mg |
| | Distilled water | Adequate amount Total volume was made to be 2 ml |
| 60 mg preparation | Hydrochloride of general formula (I) (where R¹ represents a hydroxyl group) | 60 mg |
| | Sodium hydrochloride | 16 mg |
| | Distilled water | Adequate amount Total volume was made to be 2 ml |

### Example 6

Preparation example (tablet)
A tablet containing the ingredients shown in the following Table 3 was prepared through a general method.

**[Table 3]**

| | Ingredient | Amount |
|---|---|---|
| 10 mg preparation | Hydrochloride of the general formula (I) (where R¹ represents a hydrogen atom) | 10.0 mg |
| | Crystalline cellulose | 25.0 mg |
| | Lactose | 108.5 mg |
| | Magnesium stearate | 1.5 mg |
| | Carboxymethylcellulose calcium | 5.0 mg |
| | Total | 150.0 mg |
| 20 mg preparation | Hydrochloride of the general formula (I) (where R¹ represents a hydrogen atom) | 20.0 mg |
| | Crystalline cellulose | 25.0 mg |
| | Lactose | 98.5 mg |
| | Magnesium stearate | 1.5 mg |
| | Carboxymethylcellulose calcium | 5.0 mg |
| | Total | 150.0 mg |
| 10 mg preparation | Hydrochloride of general formula (I) (where R¹ represents a hydroxyl group) | 10.0 mg |
| | Crystalline cellulose | 25.0 mg |
| | Lactose | 108.5 mg |
| | Magnesium stearate | 1.5 mg |
| | Carboxymethylcellulose calcium | 5.0 mg |
| | Total | 150.0 mg |
| 20 mg preparation | Hydrochloride of general formula (I) (where R¹ represents a hydroxyl group) | 20.0 mg |
| | Crystalline cellulose | 25.0 mg |
| | Lactose | 98.5 mg |
| | Magnesium stearate | 1.5 mg |
| | Carboxymethylcellulose calcium | 5.0 mg |
| | Total | 150.0 mg |

### Industrial Applicability

The agent for the prevention of and/or treatment for vasospasm accompanying a bypass operation, which contains as an active ingredient the compound of the present invention, can effectively treat the vasospasm which occurs in an artery bypass operation which is performed when occlusion or stenosis of an artery has occurred. Thus, the agent of the present invention is industrially useful.

## Claims

1. An agent for prevention of and/or treatment for vasospasm accompanying a bypass operation, which comprises as an active ingredient either a compound represented by the following general formula (I): 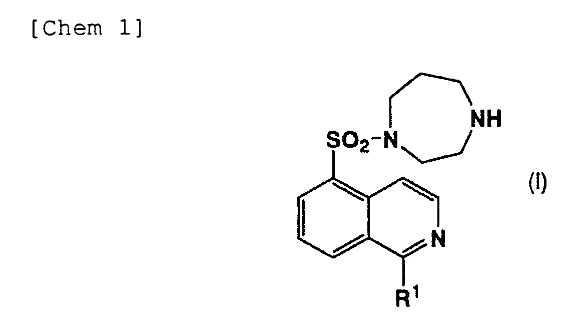 where R¹ represents a hydrogen atom or a hydroxyl group, or an acid addition salt or hydrate thereof.

2. The agent for prevention of and/or treatment for vasospasm accompanying a bypass operation according to Claim 1, wherein the bypass operation comprises coronary artery bypass grafting.

3. The agent for prevention of and/or treatment for vasospasm accompanying a bypass operation according to Claim 1 or 2, wherein the vasospasm comprises vasospasm which does not respond to a calcium antagonist and/or a nitro-compound.

4. The agent for prevention of and/or treatment for vasospasm accompanying a bypass operation according to any one of Claims 1 to 3, wherein the vasospasm occurs in a region which differs from an anastomosis region.
